# EUROPEAN PATENT APPLICATION

(11) **EP 0 941 730 A1**
(43) Date of publication of application: **15.09.1999**
(21) Application number: 99104911.5
(22) Date of filing: 12.03.1999
(51) Int. Cl.: A61F 13/58

(54) **Fastening tape**

(30) Priority: 13.03.1998 EP 98104590
(71) Applicant: MINNESOTA MINING AND MANUFACTURING COMPANY, St. Paul, Minnesota 55133 (US)
(72) Inventor: Pape, Peter c/o Minnesota mining and manuf.Co, St Paul,Minnesota 55 144-1000 (US); Tuschy, Jörg c/o Minnesota mining and manuf.Co, St Paul,Minnesota 55 144-1000 (US); Günther,Werner T.c/o Minnesota mining and manuf.Co, St Paul,Minnesota 55 144-1000 (US); Kourtidis,Konstantinos c/o Minnesota mining man.Co, St Paul,Minnesota 55 144-1000 (US)
(74) Representative: Wilhelm, Stefan

(57) **Abstract**

The invention refers to mechanical a closure tape (10) for an absorbent article, particularly for a disposable diaper (1), for the fastening of the article on the body of a person, the mechanical closure tape (10) being attachable to the outside surface (3) of the diaper (1) through its manufacturer's end (19) and comprising a backing (11) bearing an adhesive layer (12), optionally a covering film (13) and/or a stretchable elastic film (13a), a fastening means (14), optionally a fingerlift (15) and a one-sided adhesive tape (16) having a backing (16b) bearing an adhesive layer (16a), the adhesive tape (16) being attachable to the inside surface (2) of the diaper and/or to the connective portion (21) of the closure tape (10) by means of the adhesive layer (16a), by means of the adhesive layer (12) and/or by means of one or more one-sided further adhesive tapes so that the adhesive layer (16a) is at least partially exposed above the inside surface (2) of the diaper.

## Description

### Field of the invention

The present invention relates to a mechanical closure tape for an absorbent article, particularly for a disposable diaper, which
- allows to fasten the article on the body of a person during use employing a mechanical fastening means,
- allows to secure the soiled diaper after use for disposal (disposability feature), and
- is furthermore designed not to pop open when folded onto the inside surface of the diaper prior to use (anti-flagging feature).

The present invention also relates to a prelaminated mechanical closure tape in a stable roll form from which the mechanical closure tape can be cut. The present invention furthermore refers to a method of preparing said mechanical closure tape and to an absorbent article comprising said mechanical closure tape.

### Background of the invention

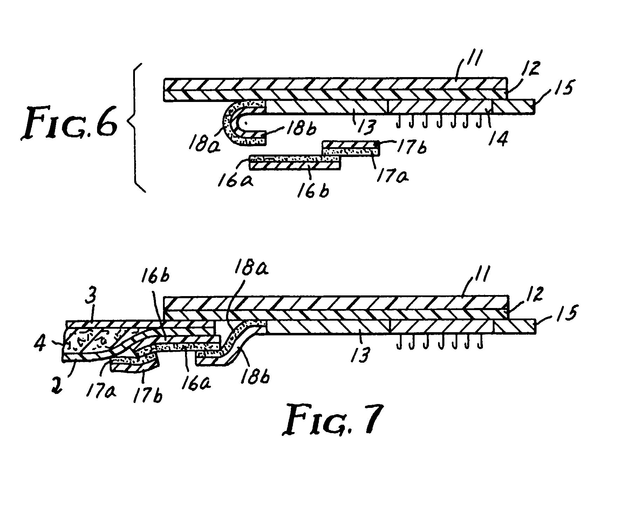

Closure tapes for absorbent articles having some or all of the above features, are described in the art. Fig. 4 of the Japanese patent (kokoku) JP 08-2,365 shows a mechanical closure tape which is attached to the outside surface of the diaper through its manufacturer's end (L₁) and which carries on its connective portion (L-L₁) a hook fastening element 6B and two small strips 6C₁ and 6C₂ of a temporary holding adhesive on both sides of the hook fastening element 6B. On folding the connective portion of the mechanical closure tape onto the inside surface of the diaper, the temporary holding adhesive strips are transferred as 6C₆ and 6C₇ onto the inside surface of the diaper as can be seen in Fig. 7 of JP 08-2,365. The adhesive strips temporarily secure the connective portion of the mechanical closure tape to the inside surface of the diaper and prevent the mechanical closure tape from popping open when handling semi-finished absorbent articles during the process of manufacturing or when storing, packaging or transporting the finished absorbent article. The property of the mechanical closure tape not to pop open is often also referred to as anti-flagging feature. When the diaper described in JP 08-2,365 is applied to the wearer's body, the connective portion of the closure tape is removed from the inner surface of the diaper leaving the two adhesive strips 6C₆ and 6C₇ behind, and it is attached by means of the hook fastening element 6B onto a complementary mechanical fastening element on the landing zone of the diaper. Mechanical fastening elements are preferred over adhesive fastening elements because mechanical fastening elements tend not to be adversely affected by skin creams or powders and can easily be released and re-closed. The mechanical closure tape of JP 08-2,365 does not provide, however, a disposal feature.

EP 0,321,232 discloses mechanical closure tapes for disposable absorbent articles, particularly diapers, comprising on their connective portion a mechanical hook fastening element. While the mechanical hook fastening element is engagable with a complimentary mechanical loop fastening element on the landing zone of the diaper and thus secures the diaper to the wearer's body during use, the exposed adhesive fastening element provides for an anti-flagging feature and can furthermore adhere to the outside surface of the diaper when it is rolled up for disposal. EP 0,321,232 does not disclose a prelaminated mechanical closure tape in a stable roll from which the mechanical closure tape can be cut.

It is pointed out in EP 0,780,109 that when applying closure tapes having fastening means on the connective portion of the closure tape only, to the front part of the diaper including the landing zone, such front part may tend to fold down thereby creating a hard transition zone between the front portion and the diaper ear to which the manufacturer's end of the closure tape is attached. The transition area may contact the wearer's skin and result in skin irritations. In order to avoid such misalignment, EP 0,780,109 discloses a two-point closure tape comprising in addition to the fastening means on the connective portion of the closure tape, an exposed adhesive fastening means on the opposite part of the closure tape which covers a part of the inside surface of the diaper. The manufacture of mechanical closure tapes according to EP 0,780,109 requires, however, strip-coating and the use of double-coated adhesive tapes.

It was therefore an object of the present invention to provide a mechanical closure tape having both a disposability and an anti-flagging feature which does not have the shortcomings of the mechanical closure tapes disclosed in the state-of-the-art and which is easy to manufacture. It was another object of the present invention to provide a prelaminated mechanical closure having both a disposability and an anti-flagging feature, in a stable roll form. It was another object of the present invention to provide a simple and economically advantageous method of preparing such prelaminated mechanical closure tape in a stable roll form. Other objects of the present invention are evident from the following description of the invention.

### Brief description of the invention

The invention relates to a mechanical closure tape 10 for an absorbent article, particularly for a disposable diaper 1, for fastening of the article on the body of a person, the mechanical fastening tape 10 being attachable to the outside surface 3 of the diaper 1 through its manufacturer's end 19 and comprising a backing 11 bearing an adhesive layer 12, optionally a covering film 13 and/or a stretchable elastic film 13a, a mechanical fastening means 14, optionally a fingerlift 15 and a one-sided adhesive tape 16 having a backing 16b bearing an adhesive layer 16a, the adhesive tape 16 being attachable to the inside surface 2 of the diaper and/or to the connective portion 21 of the closure tape 10 by means of the adhesive layer 16a, by means of the adhesive layer 12 and/or by means of one or more further one-sided adhesive tapes so that the adhesive layer 16a is at least partially exposed above the inside surface 2 of the diaper.

The invention furthermore relates to a prelaminated closure tape according to the invention coiled into a stable roll form and to an absorbent article comprising at least one closure tape according to the present invention

### Brief description of the figures

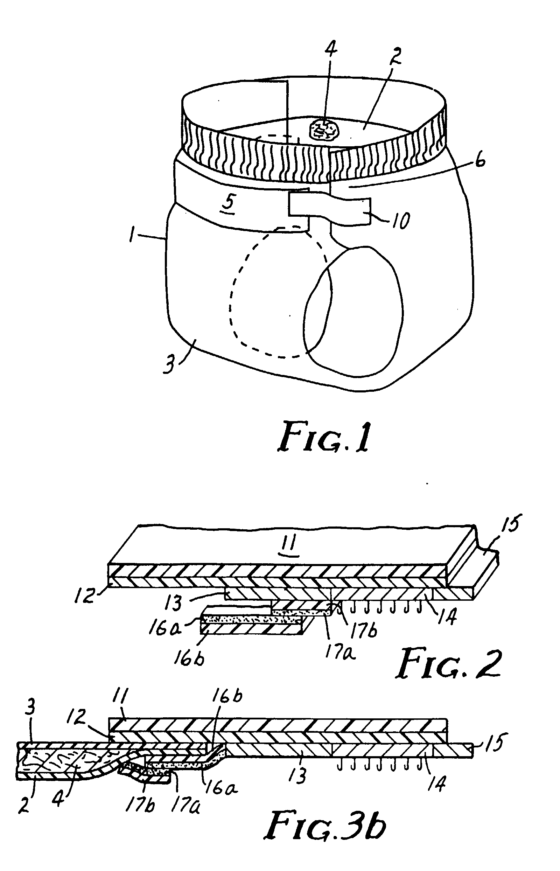

- Fig. 1: is a perspective representation of a disposable diaper 1 in a closed form, having an absorbent core 4 between an inside surface 2 and an outside surface 3, mechanical closure tapes 10 anchored to the edge portions 6 of the diaper 1 and fastened to the target area 5 on the outside surface of the diaper.
- Fig. 2: is a perspective representation of the precursor of a preferred embodiment of the mechanical closure tape 10 according to the invention comprising a backing 11 bearing a continuous adhesive layer 12, a covering film 13, a mechanical hook fastening means 14, a fingerlift 15, a first one-sided adhesive tape 16 and a second one-sided adhesive tape 17.

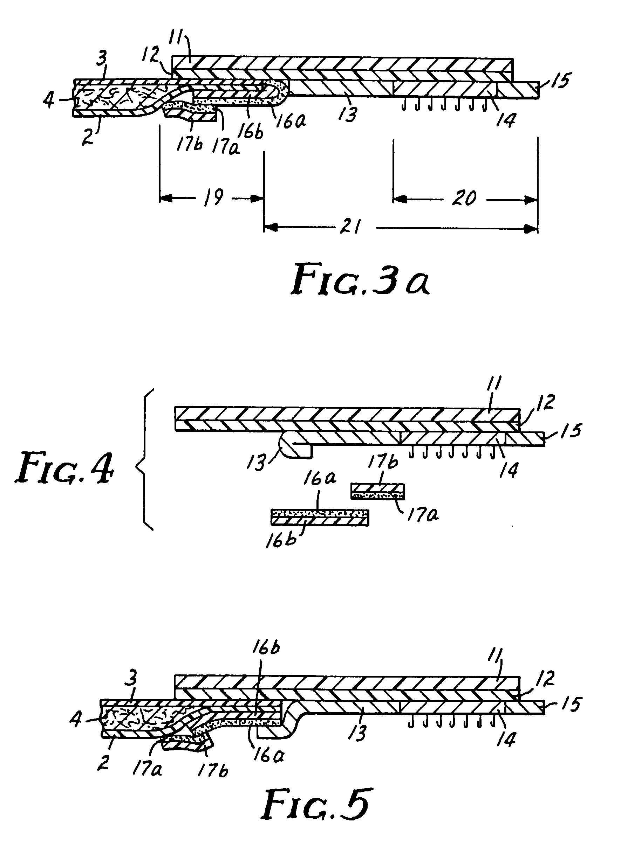

- Fig. 3a: is a cross-sectional view of the mechanical closure tape 10 obtainable from the precursor of Fig. 2 being attached to the edge portion of a diaper 6 wherein the one-sided adhesive tape 16 is distally adhered with its adhesive layer 16a to the adhesive layer 12.
- Fig. 3b: is a cross-sectional view of another preferred embodiment of a mechanical closure tape 10 which differs from the closure tape of Fig. 3a in that the one-sided adhesive tape 16 is distally adhered with its backing layer 16b to the adhesive layer 12.
- Fig. 4: is a cross-sectional view of the precursor of another preferred embodiment of a mechanical closure tape 10 according to the invention which differs from the embodiment of Fig. 2 in that the proximal end of the covering film 13 is folded back upon itself.
- Fig. 5: is a cross-sectional view of the mechanical closure tape 10 obtainable from the precursor of Fig. 4, being attached to the edge portion of a diaper.
- Fig. 6: is a cross-sectional view of the precursor of another preferred embodiment of a mechanical closure tape 10 according to the invention which differs from the embodiment of Fig. 2 in that the adhesive tape 16 is securable to the adhesive layer 12 by means of a third one-sided adhesive tape 18.
- Fig. 7: is a cross-sectional view of the mechanical closure tape 10 obtainable from the precursor of Fig. 6, being attached to the edge portion of a diaper.

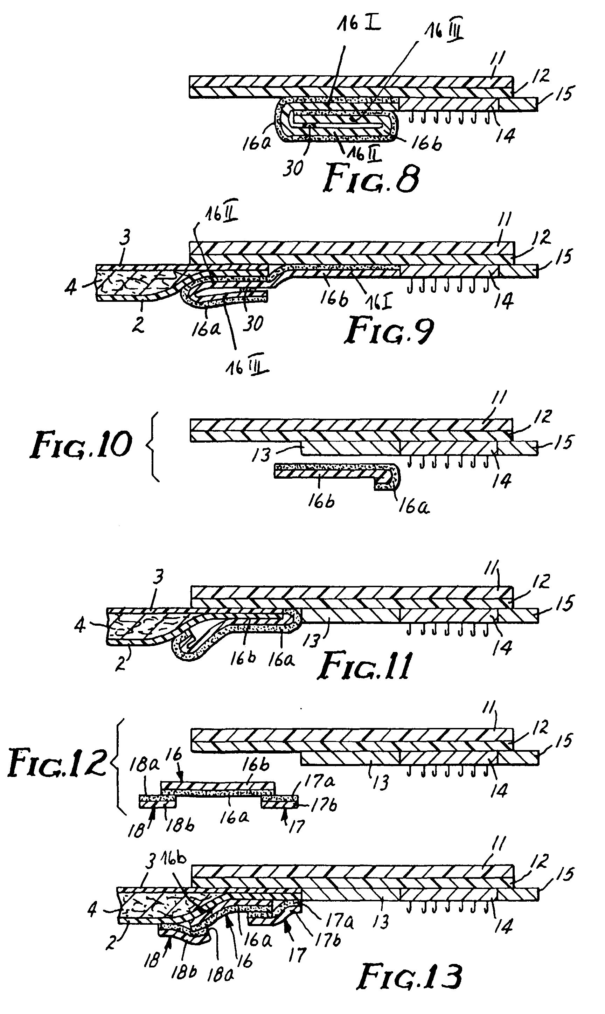

- Fig. 8: is a cross-sectional view of another preferred embodiment of a mechanical closure tape 10 according to the invention comprising a backing 11 bearing a continuous adhesive layer 12, a mechanical hook fastening means 14, a fingerlift 15 and a one-sided pressure sensitive adhesive tape 16 which is folded twice on itself so that it has a first part 16I adhered to the pressure sensitive adhesive layer 12, a second part 16II the adhesive layer 16a of which being exposed and a third part 16III between the first and second part of adhesive tape 16 so that the adhesive layer 16a of the third part 16III contacts the backing 16b of the first part 16I of the adhesive tape 16.
- Fig. 9: is a cross-sectional view of the mechanical closure tape 10 of Fig. 8 being attached to the edge portion of a diaper.
- Fig. 10: is a cross-sectional view of the precursor of another preferred embodiment of a mechanical closure tape 10 according to the invention, having a one-sided adhesive tape 16 contacting the adhesive layer 12 (broken lines) the proximal end of which being folded on itself with the adhesive layer facing outwards.

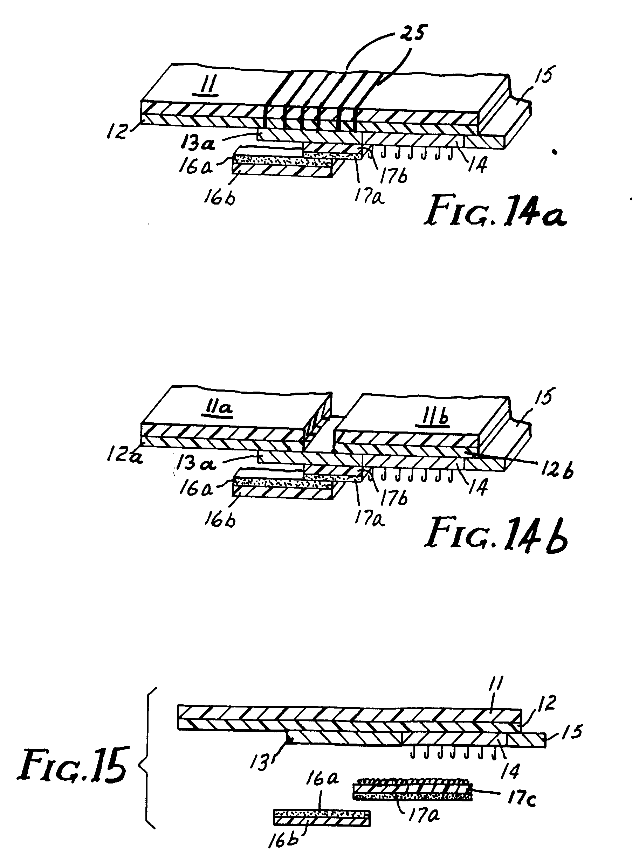

- Fig. 11: is a cross-sectional view of the mechanical closure tape 10 obtainable from the precursor of Fig. 10, being attached to the edge portion of a diaper.
- Fig. 12: is a cross-sectional view of the precursor of another embodiment of a mechanical closure tape 10 according to the present invention comprising a backing 11 bearing a continuous adhesive layer 12, a covering film 13, a mechanical hook fastening means 14 and a fingerlift 15, and a one-sided adhesive tape 16 which bears on each of its distal and proximal ends, respectively, a second and third adhesive layer 17 or 18, respectively.
- Fig. 13: is a cross-sectional view of the mechanical closure tape 10 which is obtainable by applying the precursor of Fig. 12 to the edge portion of a diaper.
- Fig. 14a: is a perspective representation of the precursor of a preferred embodiment of the mechanical closure tape 10 according to the invention comprising a backing 11 bearing a continuous adhesive layer 12, a stretchable elastic film 13a, a mechanical hook fastening means 14, a fingerlift 15, a first one-sided adhesive tape 16 and a second one-sided adhesive tape 17 whereby the backing 11 exhibits several incisions 25 in the area of the stretchable elastic film 13a, said incisions extending in vertical direction through the backing 11 and the adhesive layer 12, in machine direction over the full width of the backing 11 and in cross direction in the unstretched state over a relatively small width of, for example, 20 - 100 µm.
- Fig. 14b: is a perspective representation of the precursor of a preferred embodiment of the mechanical closure tape 10 according to the invention comprising a backing 11 consisting of two parts 11a and 11b each part bearing a continuous adhesive layer 12, a stretchable elastic film 13a, a mechanical hook fastening means 14, a fingerlift 15, a first one-sided adhesive tape 16 and a second one-sided adhesive tape 17 whereby the proximal end of the elastic film 13a is adhered through adhesive layer 12a to the distal end of the proximal part 11a of the backing 11 and the distal end of the elastic film 13a is adhered to the proximal end of the distal part 12b of the backing through adhesive layer 12b, thus creating a slit having a relatively large extension in cross direction in the unstretched state of for example, 1 to several mm.
- Fig. 15: is a perspective representation of the precursor of a preferred embodiment of the mechanical closure tape 10 according to the invention comprising a backing 11 bearing a continuous adhesive layer 12, a covering film 13, a mechanical hook fastening means 14, a fingerlift 15, a first one-sided adhesive tape 16 comprising a backing 16b bearing an adhesive layer 16 a, and a second one-sided adhesive tape 17 comprising a mechanical fastening means 17c bearing an adhesive layer 17a.

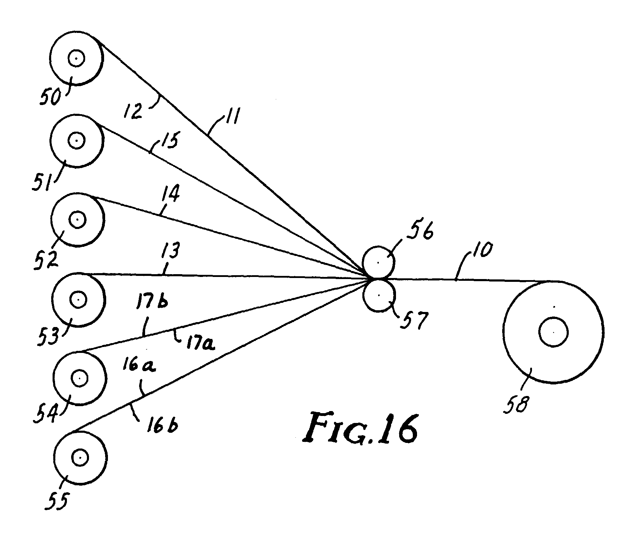

- Fig. 16: is a schematic representation of the method of manufacture of the mechanical closure tape according to Fig. 3a and a corresponding apparatus, said method comprising unwinding of the backing 11 bearing adhesive layer 12 from roll 50, of the fingerlift 15 from roll 51, of the mechanical fastening means 14 from roll 52, of the covering film 13 from roll 53, of the first and second one-sided adhesive tapes 16 and 17, respectively, from rolls 54 and 55, respectively, passing the above component in the order given through two laminator rolls 56 and 57, and winding up the resulting mechanical closure tape on roll 58.

### Detailed description of the invention

The mechanical closure tapes 10 of the present invention are useful and beneficial when attached to an absorbent article and, in particular, to a disposable absorbent article. As used herein, the term "disposable absorbent article" refers to articles which are placed against or in proximity to the body of the wearer to absorb and contain the various exudates discharged from the body and which are intended to be disposed of after single use (i.e., they are not intended to be laundered or otherwise restored or reused).

A preferred embodiment of the disposable absorbent article of the present invention is a diaper. As used herein, the term "diaper" refers to a garment generally worn by infants or incontinent persons that is drawn up between the legs and fastened about the waist of the wearer.

The term "mechanical closure tape" as used herein denotes a closure tape having a mechanical fastening means 14 for securing the absorbent article to the wearer's body during use. The term "manufacturer's end" refers to the part 19 of the closure tape which is attached to the outside surface 3 of the diaper 1. The term "connective portion" 21 refers to the part of the closure tape 10 which forms the free end of the closure tape 10 The term "user's end" refers to the part 20 of the closure tape 10 comprising the mechanical fastening means 14 and the fingerlift 15. These parts of the closure tape are shown in Fig. 3a.

Fig. 1 is a partially cut-away perspective representation of a disposable diaper 1 in a closed form. The diaper comprises an absorbent core 4 between an inside surface 2 and an outside surface 3. The absorbent core 4 may be any means which is generally compressible, conformable, non-irritating to the wearer's skin, and capable of absorbing and retaining liquids and certain body exudates.

The outside surface 3 of the diaper is impervious to liquids and is preferably manufactured from a thin plastic film, although other flexible, liquid impervious materials may also be used. The outside surface 3 prevents the exudates absorbed and contained in the absorbent core 4, from soiling articles which contact the diaper 1 such as bedsheets and undergarments.

The inside surface 2 of the diaper is compliant, soft-feeling, and non-irritating to the wearer's skin. Further, the inside surface 2 is liquid pervious permitting liquids to readily penetrate through its thickness. A suitable inside surface 2 may be manufactured from a wide range of materials such as porous foams, reticulated foams, apertured films, natural fibers (e.g., wood or cotton fibers), synthetic fibers (e.g., polyester or polypropylene fibers) or from a combination of natural and synthetic fibers. Preferably, it is made of a hydrophobic material to isolate the wearer's skin from liquids retained in the absorbent core 4. A suitable inside surface 2 may be, for example, a spunbond or carded polypropylene nonwoven of approximately 15-25 g/m².

The absorbent core 4 may be secured to the outside surface 3 by means of, for example, pressure-sensitive adhesives, hot melt adhesives or other adhesives, ultrasonic bonding or heat/pressure sealing. The outside surface 3 and the inside surface 2 may be joined to each other directly or indirectly by using an intermediate fixing member to which the outside surface 3 and the inside surface are affixed. The inside surface 2 and the outside surface 3 may be associated together by various means comprising, for example, pressure-sensitive adhesives, hot melt adhesives or other adhesives, ultrasonic bonding and/or heat and/or pressure.

The above description of the diaper 1 is meant to be explanatory only and not limiting. Further details on diapers and their construction are described in literature and may be taken, for example, from EP 0,529,681, US 4,036,233, EP 0,487,758, WO 96/10,382, US 3,800,796, EP 0,247,855 or US 4,857,067.

The mechanical closure tape 10 is secured to the edge portion 6 of the diaper 1 through its manufacturer's end 19 whereby several configurations are possible. In the embodiment of Fig. 13 the one-sided adhesive tape 16 is attached to the inside surface 2 of the diaper 1 only and has no connection to the connective portion 21 of the mechanical closure tape 10. In the embodiment of Fig. 3a the distal end of the adhesive layer 16a of the one-sided adhesive tape 16 is adhered to the adhesive layer 12 so that the mechanical closure tape 10 is attached both to the inside surface 2 and outside surface 3 of the diaper. The anchoring modes shown in Fig. 5 and 8 allow for especially reliably securing the mechanical closure tape 10 to the edge portion 6 of the diaper 1.

Anchoring modes other than those shown in the figures are possible. In an alternative construction, the backing 11 optionally comprising an adhesive layer 12, may be bonded at the manufacturer's end between the outside surface 3 and the inside surface 2 of the diaper.

In the closed state of the diaper 1, the mechanical fastening means 14 of the mechanical closure tape 10 is attached to the target area 5. The target area 5 may be formed of an additional strip which is attached to the outside surface 3 of the diaper 1 in such a manner that the size of the diaper or garment may be adjusted in accordance with the size of the user. The target area can comprise one or more such strips and could also form the entire outside surface 3 of the diaper. The outer surface of the target area 5 is selected to engage with the mechanical fastening means 14 of the mechanical closure tape 10 in an overlapping configuration to provide a secure side closure. For example, in case the fastening means 14 comprises a hook material, the outer surface of the target area will comprise any suitable material which interlocks with hook material such as, for example, woven or non-woven fabrics.

The absorbent article and, in particular, the disposable diaper 1 of the present invention differs from prior art contoured absorbent articles in that it comprises a novel mechanical closure tape 10. Fig. 2 shows a cross-sectional view of the precursor of a preferred embodiment of the mechanical closure tape.

The precursor of the mechanical closure tape 10 shown in Fig. 2 comprises a backing 11 which is preferably selected from a group of materials which is essentially non-elastic and imparts a desirable stiffness to the mechanical closure tape 10.

The backing 11 is especially preferably selected from a group of materials comprising polypropylene, polyvinylchloride, polyethylene terephthalate, polyethylene, polyolefin copolymers or blends of polyolefins such as, for example, blend of polypropylene and LPDE (low density polyethylene), non-woven and foamed materials. The thickness of the backing is preferably between 50 µm and 500 µm, and more preferably between 80 µm and 150 µm.

The backing 11 bears an adhesive layer 12 which preferably extends over the whole length of the mechanical closure tape 10 but may also cover only part of the backing 11. It is, for example, possible that the covering film 13 and the adhesive layer 12 in the area of the covering film 13, are omitted.

The adhesive of the adhesive layer 12 is preferably selected to permanently attach the manufacturer's end 19 of the backing 11 to the outside surface 3 of the diaper 1 during its manufacture so that the closure tape is not removed from the outside surface 3 of the diaper when the diaper 1 is used and opened or closed several times. Likewise, the adhesive of adhesive layer 12 is selected to permanently adhere the covering film 13, if present, the fastening means 14 and the fingerlift 15, if present, to the backing 11. The adhesive useful for adhesive layer 12 comprises pressure-sensitive adhesives including pressure-sensitive hot-melt adhesives and non-pressure-sensitive adhesives. The pressure-sensitive adhesives which are preferred, are preferably selected to exhibit a 90° peel adhesion from a polyethylene surface as measured according to a slightly modified test method FTM2, FINAT test method no. 2, FINAT Technisches Handbuch, 4. Auflage (1995), pp. 6-7 of at least 3.5 N/cm, more preferably of at least 5 N/cm and especially preferably of at least 6 N/cm. The test method used deviates from FTM2 in that a polyethylene film STA-211 (thickness 150 µm, adhered to a stainless steel substrate with a double-sided adhesive film) is used as the test substrate and in that the peel adhesion measurement is performed 2 min alter applying the adhesive strip to the substrate. The pressure-sensitive adhesive furthermore preferably exhibits a high value of static shear as measured according to a slightly modified test method FTM8, FINAT test method no. 8, FINAT Technisches Handbuch, 4. Auflage (1995), pp. 15-16, of at least 100 min, more preferably of at least 300 min and especially preferably of at least 500 min to ensure that the diaper does not inadvertently come loose from the wearer's body. The test method used deviates from FTM8 in that a weight of 500 g is used instead of 1000 g, that the temperature is 38 ± 2°C instead of 23 ± 2°C and that the test specimen comprises a polyethylene film (thickness 40 µm) as the test substrate and a propylene backing with a thickness of 110 µm with the adhesive to be tested attached to it as the adhesive strip which bears the weight.

Suitable pressure-sensitive adhesives include rubber-based adhesives (also called rubber-resin adhesives) which comprise natural or synthetic rubber materials and typically also tackifying resins in order to render the rubber materials tacky. Preferred examples of rubber-based pressure-sensitive adhesives are the polystyrene polyisoprene block copolymers tackified with synthetic polyterpene resins or hydrocarbon resins. Suitable pressure-sensitive adhesives furthermore include acrylate-based pressure-sensitive adhesives such as, for example, those disclosed in US Re 24,906 or US 4,710,536. The adhesives mentioned above are given only by way of example, and the person skilled in the art can select other adhesives known in the state of the art without any inventive effort. The thickness of the adhesive layer 12 preferably is between 10 and 200 µm and more preferably between 20 and 100 µm.

The exposed surface of the backing 11 preferably has release properties and/or is treated with a release coating so that the backing 11 bearing the adhesive layer 12 can be wound up to a roll without requiring a release liner. The release coating may be any of the known materials used for their release properties for adhesives. Preferred types are silicones and modified silicones, the modification including both copolymerization of silicones with other non-release chemical agents or by adding non-silicone materials to the silicone coating solution prior to application to the release base paper. Other release agents such as polyethylene, fluorocarbons and polyvinyl octadecyl carbamate may also be used. The choice of release coating is dependent on the tack, adhesion level, and chemical nature of the adhesive layer 12.

The covering film 13, if present, is preferably selected from a group of materials comprising polyolefin homopolymers or copolymers such as, for example, homopolymers or copolymers of ethylene and/or propylene, polyesters or blends comprising such polymers.

The covering film 13 may also be selected from the group of stretchable elastic materials and is then designated as stretchable elastic film 13a which, if present, in combination with one or more incisions (also termed as slits) 25 vertically extending from the surface of the backing 11 through the adhesive layer 12 to the upper surface of the stretchable elastic film 13a, renders the closure tape 10 elastically stretchable in cross direction (CD) thus increasing the fit and the comfort of the absorbent article. For the purposes of the present invention a material is considered to be stretchable elastic in a given direction if a sample of the material (dimensions 25 mm x 18 mm x 0.1 mm) is stretchable in that direction from a first and generally relaxed length (no external tensional forces applied) to a second or expanded length which is at least two times the first length and upon relaxation, will retract to a third length which is no greater than 1.1 times the first length with these figures referring to the second tension-relaxation cycle. Materials which irreversibly deform when a sample of the material (dimension 25 mm x 18 mm x 0.1 mm) is stretched in a given direction from a first and generally relaxed virgin state (no external tension force, no cycle comprising application of tension force and release of tension force applied yet) to a second or expanded state with a length of 1.5 times of the first length so that upon release of the tension force the material remains in the expanded state or retracts to a third state that is at least 1.25 of the first length, or which breaks before being stretched to a length of 1.5 times the original length, are termed as non-elastic with respect to the given direction.

Upon stretching, the stretchable elastic film 13a exerts a restoring force onto the joint formed between fastening means 14 of the mechanical closure tape 10 and target area 5 of the diaper 1 which is advantageous in order to reliably secure, for example, the diaper 1 around the wearer's waist. The cross direction corresponds to the longitudinal symmetry axis of the closure tape 10 and is orthogonal to the machine direction or lateral symmetry axis of the tape.

The elastic film 13a may be made from a group of materials comprising essentially isotropic or essentially anisotropic materials. Useful elastic materials preferably exhibit an elongation at break as measured according to ASTM D 882 in the preferred direction of stretchability of at least 700 % or more and, more preferably, of more than 850 %.

Preferred essentially isotropically elastic materials include elastomeric polyurethane materials such as, for example, those available under the trademark ESTANE from B.F. Goodrich & Co., natural or synthetic rubber materials such as, for example, ethylene-propylene-dien copolymers (EPDM), styrene-butadiene-styrene block copolymers (SBS), styrene-(ethylenebutylene)-styrene block copolymers (SEBS) such as, for example, those available from the Shell Chemical Company under the trademark KRATON G like, for example, KRATON G-1657. Other elastomeric materials which may be used to form the elastic sheet 13 include elastomeric polyamide materials such as, for example, those available under the trademark PEBAX from the Rilsan Company and elastomeric polyester materials such as, for example, those available under the trade designation Hytrel from E.I. DuPont De Nemours & Company.

When attaching an essentially isotropic elastic material to the backing 11, the elasticity of the laminate in the machine direction will be essentially determined by the non-elastic and/or non-extensible, respectively, backing 11. Therefore essentially isotropically elastic materials can be used for the preparation of the closure tapes 10 of the present invention without rendering it unstable or wobbly in the machine direction.

Preferred essentially anisotropic elastic materials include extruded blends of a block copolymer elastomer portion and a polyolefin polymer portion, respectively, blended in a ratio from 10:1 to 0.4:1, wherein the block copolymer elastomer portion is formed of A blocks and B blocks, the A blocks being predominantly formed of monoalkenyl arenes and the B blocks predominantly of conjugated dienes and wherein the polyolefin polymer portion is comprised predominantly of an inelastic fiber forming polyolefin polymer, copolymer or blend. Such materials are described in copending US patent application serial no. 08/720,794 filed by the present applicant on October 4, 1996. JP 51-86,611 discloses extrusion of a blend of an ABA block copolymer with polystyrene where the polymers are exemplified as blended at a ratio of from 50 to 99 percent block copolymer to 1 to 50 percent polystyrene. The resulting material exhibits anisotropic behaviour. Other essentially anisotropically elastic materials which are useful in the present invention are described, for example, in US 5,344,691, US 5,501,679 and US 5,354,597.

Anisotropically elastomeric materials which are useful in the present invention preferably exhibit a ratio of the F10 force required to stretch a sheet of the elastomeric material (dimensions 20 x 25 mm) by 10% into the machine direction over the F10 forces required in the cross direction, of at least 1.5, more preferably of at least 2.0 and especially preferably of at least 2.5.

Especially preferred stretchable elastic materials are disclosed in EP 0,891,760 and in the co-pending patent applications filed by the present applicant as PCT/US 97/18,416 and as U.S. S.N. 09/055,173 (filing date April 3, 1998).

The stretchable elastic film 13a can be attached to the backing 11 by the adhesive layer 12, and one or more slits or incisions 25 are applied in the area of the stretchable elastic film 13a extending from the outside surface of the backing 11 through the adhesive layer 12 to the upper surface of the elastic film 13a contacting the adhesive layer 12.

The thickness of the covering film 13 or the stretchable elastic film 13a, respectively, preferably is between 50 and 100 µm and more preferably is between 10 and 40 µm. The mechanical closure tape 10 of the present invention can also comprise a combination of one or more covering films 13 and one or more stretchable elastic films 13a.

The extension of the incisions or slits 25 in the unstretched state in the cross direction may vary from about 10 µm to several mm. When using several slits in a construction according to Fig. 14a, the width of the slits in cross direction preferably is less than 100 µm, more preferably less than 50 µm and especially preferably between 10 and 30 µm. It was found that if the extension of the slits is less than approximately 25 µm, essentially no adhesive will be squeezed through the incisions or slits 25 on stretching and relaxing the closure tape 10 for several times, for example, for 3-5 times, so that the outer surface of the backing 11 remains essentially clean and is not contaminated with adhesive during use. When using a construction corresponding to Fig. 14b with one or more relatively broad slits, the width of the slits in cross direction preferably is several 100 µm to several mm and, in particular, between 500 µm and 3 mm.

In the machine direction, the slits or incisions 25 may extend over the full width of the tape as is shown, for example, in Figs. 14a and 14b, or over part of the width only as is shown, for example, in Fig. 10. Further details on the geometry of the slits can be taken, for example, from EP 0,891,760.

The mechanical fastening means 14 is the male or female part, respectively, of a mechanical closure system comprising two interlocking means. The closure system may be, for example, a hook-and-loop system. The fastening means 14 may comprise the hook fastener or the loop fastener means, respectively, but it preferably comprises the hook fastener means. The hook fastener means may have any shape such as hooks, "T's" or any other shape as are well known in the art. The hook fastener material may be manufactured from a wide range of materials including nylon, polyester, polyolefins or any combination of these. A preferred hook material comprises a base and a plurality of engaging elements which comprise a stem supported at the base and an enlarged head which is positioned at the end of the stem opposite of the base. Such hook material is commercially available from 3M Company, St. Paul, U.S.A. under the trade designation KN-2396.

The loop material may be comprised of woven or non-woven fabric or any other suitable material which interlocks with the contemplary hook fastener material. A suitable loop fastening material comprises a number of fiber loops projecting from a woven backing such as the Knitted Loop Tape EKLT-1112, commercially available from 3M Company, St. Paul, U.S.A. The mechanical fastening means 14 preferably has an extension in cross-direction (i.e. in the direction of the long axis of the mechanical closure tape 10) of between 10 and 40 mm, and more preferably of between 15 and 30 mm.

The fingerlift 15 may optionally be attached to the distal end of the connective portion 21 to allow for easier handling of the mechanical closure tape 10. The fingerlift 15 may partly extend over the distal end of the backing 11 as is shown, for example, in Fig. 2. In another embodiment, the backing 11 may extend over the distal end of the adhesive layer 12 and, optionally, be folded back onto the adhesive layer 12 to provide for a fingerlift 15. Alternatively the fingerlift 15 may be attached to the backing 11 by different welding techniques such as, for example, ultrasonic welding. The fingerlift 15, if present, preferably has an extension in cross-direction of typically between 3 and 10 mm, a thickness of between 25 and 200 µm and it is preferably prepared from a group of materials comprising polymer films and non-wovens.

The mechanical closure tape 10 according to the present invention furthermore comprises a one-sided adhesive tape 16 having a backing 16b and an adhesive layer 16a. The one-sided adhesive tape 16 is applied to the inside surface 2 of the diaper and/or to the connective portion 21 of the mechanical closure tape 10 by means of the adhesive layer 16a, by means of adhesive layer 12 and/or by means of one or more one-sided further adhesive tapes so that the adhesive layer 16a faces away from the inside surface 2 of the diaper and is at least partially exposed above the inside surface of the diaper.

Mounting of the one-sided adhesive tape 16 to the inside surface 2 of the diaper 1 and/or the connective portion 21 of the mechanical closure tape 10 can be effected in various ways, and some preferred, non-limiting constructions are schematically shown in Figures 2 - 13.

Fig. 2 shows the precursor of the mechanical closure tape of Fig. 3a. A laminate is provided comprising one-sided adhesive tape 16 and a further one-sided adhesive tape 17 having an adhesive layer 17a and a backing 17b. The distal end of the adhesive layer 16a of tape 16 is laminated to the adhesive layer 17a of tape 17 in an overlapping fashion so that the adhesive tape extends over the distal edge of adhesive tape 16. The closure tape 10 is obtained by laminating the proximal end of the adhesive layer 16a of tape 16 to adhesive layer 12 adjacent to the proximal end of the cover film 13.

Fig. 3a shows the closure tape 10 obtained from the precursor of Fig. 2 being applied to the edge portion 6 of a diaper 1. The laminate comprising one-sided adhesive tapes 16 and 17, respectively, which is distally adhered by adhesive layer 16a to the adhesive layer 12, has been folded back so that the laminate is proximally adhered to the inside surface 2 of the diaper by means of adhesive layer 17a. The adhesive layer 16a faces away from the inside surface of the diaper and is exposed.

During the manufacture of the diaper 1 and/or when packaging and storing it prior to use, the connective portion 21 of the mechanical closure tape 10 as shown in Fig. 3a is usually folded onto the inside surface 2 of the diaper 1 thereby contacting the exposed adhesive layer 16a of one-sided adhesive tape 16. In the embodiment of Fig. 3a which is preferred, the connective portion 21 contacts the adhesive layer 16a via the covering film 13. The adhesive used in the adhesive layer 16a is selected so that
- the connective portion 21 can be releasably adhered to adhesive layer 16a to provide for an anti-flagging feature, and
- the adhesive layer 16 can be reliably adhered to the backside surface 3 of the diaper to provide for a disposability feature.

The adhesion behavior is governed by the nature of the adhesive layer 16a and by the surface properties of the covering film 13 or the backside surface 3 of the diaper 1, respectively. When using, for example, an aggressive pressure sensitive adhesive in order to allow for reliably securing of the exuded diaper 1, the covering film 13 typically comprises a film having appropriate release surface properties and/or a release coating. When using, for example, an aggressive pressure sensitive adhesive material for adhesive layer 16a, which is selected from the pressure sensitive adhesive materials described above for use in adhesive layer 12, the covering film 13 preferably has a release coating. Suitable release coatings may be selected from the group of release materials described above for the backing 11. The person skilled in the art can modify the nature of the adhesive material of adhesive layer 16a and/or the covering film 13, respectively, or the surface release properties of the covering film 13 in order to provide the desired adhesion behavior between adhesion layer 16a and the covering film 13 or the backside surface 3 of the diaper 1, respectively.

The backing layer 16b of the one-sided adhesive tape 16 may be manufactured from a wide range of materials such as polymer films or paper films. The backing is preferably selected from a group of materials comprising polypropylene, polyvinylchloride, polyethylene terephthalate, polyethylene, polyolefin copolymers or blends of polyolefins such as, for example, a blend of polypropylene and LDPE (low density polyethylene), non-wovens and foamed materials.

The thickness of the backing 16b preferably is between 50 µm and 300 µm and more preferably between 50 µm and 250 µm. The thickness of the adhesive layer 16a preferably is between 30 µm and 150 µm and more preferably between 30 µm and 100 µm. The extension of the one-sided adhesive tape 16 in cross-section is preferably selected so that the area of exposed adhesive above the inside surface 2 of the diaper 1 is between 10 mm² and 30 mm² and more preferably between 12 and 20 mm². The surface of the backing 16 which is opposite to the surface bearing the adhesive layer 16a, preferably has release properties and/or is treated with a release coating so that the adhesive tape 16 can be wound up to a roll without requiring a release liner.

As can be seen from Fig. 3a, the one-sided adhesive tape 16 is adhered to the inside surface 2 of the diaper 1 by means of a second one-sided adhesive tape 17 having an adhesive layer 17a and a backing 17b. Adhesive tape 17 is adhered with its adhesive layer 17a to the proximal end of adhesive layer 16a (Fig.3a), and it extends beyond the proximal edge of adhesive tape 16. The portion of adhesive tape 17 overlapping with adhesive tape 16, preferably has a width in cross-direction of between 3 and 10 mm, and the width of the extending portion of the adhesive tape 17 in cross-direction likewise preferably is between 3 and 10 mm in order to allow for secure anchoring of adhesive tape 16.

The material of the adhesive layer 17a, of the backing 17b and the thicknesses of these layers are preferably chosen using the guidelines given above for the corresponding layers of adhesive tape 16.

The embodiment of the mechanical closure tape 10 which is shown in Fig. 3b differs from the embodiment of Fig. 3a in that the one-sided adhesive tape 16 is distally adhered to the adhesive layer 12 by means of the backing 16b. The closure tape 10 of Fig. 3b can be obtained from the precursor of Fig. 2 by appropriately choosing the orientation of the laminate comprising one-sided adhesive tapes 16 and 17. In case the outside surface of the backing 16b (which surface is opposite to the surface of backing 16b adhered to the adhesive layer 16a) comprises a release coating, such release coating preferably is not present at least at the distal end of the adhesive tape 16 to allow for reliably securing the backing 16b to the adhesive layer 12. This can be obtained, for example, by strip-coating a release coating onto the outside surface of the backing 16b. It is also possible not to apply a release coating to the outside surface of 16b and cover the adhesive layer 16a with a release liner in order to allow for winding up the one-sided adhesive 16 in a roll for storage prior to lamination.

The precursor of the embodiment of the mechanical closure tape 10 according to the present invention which is shown in Fig. 4, differs from the precursor of Fig. 2 only in that the proximal end of the covering film 13 is folded back onto itself Fig. 5 shows the mechanical closure tape 10 which is obtained from the precursor of Fig. 4, being applied to the inside surface 2 of the edge portion 6 of the diaper 1. The folded part of the covering film 13 has been unfolded and overlaps with the distal end of adhesive layer 16a by preferably 3 - 10 mm and more preferably 3 - 5 mm. The configuration of Fig. 5 provides for an especially reliable and strong attachment of the mechanical closure tape 10 to the diaper 1 which is usually termed as shear mode or Y mode type of attachment.

The precursor of the embodiment of the mechanical closure tape 10 of the present invention which is shown in Fig. 6, provides a shear mode or Y mode type of attachment by using an additional one-sided adhesive tape 18 which is distally adhered with its adhesive layer 18a to adhesive layer 12 or, alternatively, to covering film 13 (not shown). In the embodiment of Fig. 6 it is also possible to use a polymeric film instead of the one-sided adhesive tape 18. Such polymeric film is preferably selected from the group of materials specified for backing 16b above. In the precursor of Fig. 6 such polymeric film is distally secured to adhesive layer 12. Adhesive tape 18, if present, comprises a backing 18b and an adhesive layer 18a. The materials used for such layers and their respective thicknesses are selected following the guidelines given above for adhesive tape 17. Similarly to adhesive tape 17, the proximal end of adhesive tape 18 is applied to the distal end portion of adhesive layer 16a with its adhesive layer 18a in an overlapping fashion so that it extends over the distal edge of adhesive tape 16 (Fig.7). The dimensions of the overlapping and extending portion, respectively, of adhesive tape 18 are selected following the guidelines given above for adhesive tape 17. Fig. 7 shows the mechanical closure tape 10 which is obtainable from the precursor of Fig. 6, being applied to the inside surface 2 of the edge portion 6 of the diaper 1. One-sided adhesive tape 18 proximally secures the distal end of adhesive tape 16 and it is distally adhered to adhesive layer 12 or, alternatively, covering film 13 (not shown).

The embodiments of the mechanical closure tape 10 shown in Fig. 8 and 10, respectively, differ from the embodiments of Figs. 2, 4 and 6, respectively, in that no additional one-sided adhesive tape is required other than one-sided adhesive tape 16.

The embodiment of the closure tape 10 shown in Fig. 8 comprises a one-sided adhesive tape 16 which is folded twice on itself so that it has a first part 161 which is adhered to the pressure sensitive adhesive layer 12, a second part 16II the adhesive layer 16a of which being exposed and a third part 16III between the first and the second part of adhesive tape 16 so that the adhesive layer of the third part 16III contacts the backing 16b of the first part of the adhesive tape 16. Fig. 9 shows that the one-sided adhesive layer 16a is partly unfolded in order to adhere the adhesive layer 16a of the second part 16II of adhesive tape 16 to the inside surface 2 of the diaper 1. The third part 16III of the adhesive tape 16 remains folded onto the second part so that the adhesive layer 16a of the third part 16III is exposed and provides for a disposability and anti-flagging feature.

The dimensions of the one-sided adhesive tape 16 in the embodiment of Fig. 8, 9 are preferably selected so that the contact area between adhesive layer 12 and adhesive layer 16a has a width in cross-direction of between 5 and 50 mm and more preferably of between 10 and 25 mm, and so that the exposed third part of the adhesive layer 16a over the inside surface 2 of the diaper 1 preferably is between 5 and 50 mm and more preferably between 10 and 25 mm wide.

In the folded state of Fig. 8, the pressure sensitive adhesive layer 16a of the third part 16III of the one-sided pressure sensitive adhesive tape 16 adheres to the backing 16b of the first part 16I of tape 16 thus releasably securing tape 16 in the folded state. In the unfolded state of Fig. 9, the third part 16III of the adhesive tape 16, the adhesive layer 16a of which is exposed, tends to remain flat and to not pop apart because the third part 16III of the one-sided adhesive tape 16 is strongly and flatly folded onto the backing 16b of the second part 16II of the adhesive tape 16. Alternatively, the backing 16b of the third part 16III of adhesive tape 16 may be adhered to the backing 16b of its second part 16II using, for example, a small spot 30 of an adhesive, wax or the like. It is also possible to attach the third part 16III of adhesive tape 16 to its second part 16II by ultrasonic spot bonding or heat spot bonding.

Fig. 10 shows the precursor of a mechanical closure tape 10 according to the present invention. The one-sided adhesive film 16 is distally folded upon itself and the precursor is converted to the corresponding closure tape 10 by laminating the proximal end of the adhesive layer 16a to the adhesive layer 12 adjacent to the proximal end of covering film 13.

Fig. 11 shows the closure tape 10 obtainable from the precursor of Fig. 10, being applied to the edge portion 6 of a diaper 1. The one-sided adhesive tape 16 is distally secured to the adhesive layer 12 by its adhesive layer 16a, and it is proximally adhered to the inside surface 2 of the diaper 1 by means of the adhesive layer 16a of the folded part of adhesive tape 16 (which part corresponds to the distal part of adhesive tape 16 in Fig. 10). The width of the folded proximal end part of adhesive layer 16a in Fig. 11 preferably is between 2 and 20 mm in order to provide for a reliable, secure bonding between the closure tape 10 and the inside surface of the diaper.

The one-sided adhesive tape 16 can also be applied to the inside surface 2 of the diaper by means of two further one-sided adhesive tapes 17 and 18 as is shown in Fig. 12 and 13. This embodiment is, however, less preferred because the connective portion 21 of the closure tape 10 is not connected to the inside surface of the diaper 1. This type of bonding is often referred to as a peel mode type of attachment.

Fig. 14a shows the precursor of a mechanical closure tape 10 according to the present invention which differs from the precursor of Fig.2 in that the covering film 13 is replaced with a stretchable elastic film 13a and that the backing 11 exhibits several incisions 25 in the area of the stretchable elastic film 13a, said incisions extending in vertical direction through the backing 11 and the adhesive layer 12, in machine direction over the full width of the backing 11 and in cross direction in the unstretched state over a small width of for example, 10 - 200 µm or preferably 20 - 100 µm. Using a stretchable elastic film 13a in combination with one or more incisions 25 allows for elastic stretching of the mechanical closure tape elastic in cross direction.

Fig 14b shows another embodiment of an elastically stretchable mechanical closure tape 10 according to the present invention. In this embodiment, the backing 11 comprises two parts 11a and 11b each part bearing an adhesive layer 12a and 12b, respectivly. The mechanical closure tape 10 further comprises a stretchable elastic film 13a which connects the two parts 11a and 11b of the backing whereby the proximal end of the elastic film 13a is adhered through adhesive layer 12a to the distal end of the proximal part 11a of the backing 11 and the distal end of the elastic film 13a is adhered to the proximal end of the distal part 11b of the backing through adhesive layer 12b. The slit obtained between the distal end of the proximal part 11a and the proximal end of the distal part 11b, respectively, of the backing can exhibit a broad range of widths in cross-direction of, for example, several hundred µm to several nun and preferably of 500 µm to 3 mm. It is also possible that the backing comprises more than two parts which are correspondingly connected by two or more parts of elastic films 13a.

The stretchable elastic film 13a may comprise, if desired or necessary, a release coating analogously applying the considerations discussed for the covering film 13 above. Suitable release coatings may be selected from the group of release materials described above for the backing 11. The person skilled in the art can modify the nature of the adhesive material of adhesive layer 16a and/or the elastic film 13a, respectively, or the surface release properties of the elastic film 13a in order to provide the desired adhesion behavior between adhesion layer 16a and the elastic film 13a or the backside surface 3 of the diaper 1, respectively.

Fig. 15 is a perspective representation of the precursor of a preferred embodiment of the mechanical closure tape 10 according to the invention comprising a backing 11 bearing a continuous adhesive layer 12, a covering film 13, a mechanical hook fastening means 14, a fingerlift 15, a first one-sided adhesive tape 16 comprising a backing 16b bearing an adhesive layer 16 a, and a second one-sided adhesive tape 17 comprising a mechanical fastening means 17c bearing an adhesive layer 17a. The mechanical fastening means 17c is selected so that it is engagable with the mechanical fastening means 14. If, for example, the mechanical fastening means 14 and 17c, respectively, form a hook-and-loop system with the mechanical fastening means 14 being the hook fastening means, the mechanical fastening means 17c will provide the corresponding loop fastening means, and vice versa.

Hook fastener materials which are suitable for use as mechanical fastening means 17c may be manufactured from a wide range of materials including nylon, polyester, polyolefins or any combination of these. A preferred hook material comprises a base and a plurality of engaging elements which comprise a stem supported at the base and an enlarged head which is positioned at the end of the stem opposite of the base. Such hook material is commercially available from 3M Company, St. Paul, U.S.A. under the trade designation KN-2396. Suitable loop materials may be comprised of woven or non-woven fabric or any other suitable material which interlocks with the contemplary hook fastener material. A suitable loop fastening material comprises a number of fiber loops projecting from a woven backing such as the Knitted Loop Tape EKLT-1112, commercially available from 3M Company, St. Paul, U.S.A.

When attaching the mechanical closure tape 10 obtained from the precursor of Fig. 15, to the edge portion 6 of a diaper 1 and folding the free end 21 of the closure tape 10 onto the inside surface 2 of the diaper 1, the mechanical fastening means 14 and 17c interlock with each other thus providing an additional anti-flagging feature. The mechanical closure tape 10 obtained from the precursor according to Fig. 15 thus reliably prevents the diaper 1 when folded onto the inside surface 2, from popping open. The embodiment of Fig. 15 is therefore preferred. The mechanical fastening means 17c preferably has an extension in cross-direction (i.e. in the direction of the long axis of the mechanical closure tape 10) so that at least part of it overlaps with the mechanical fastening means 14.

The closure tape 10 of the present invention can be prepared in an easy and reliable manner. Fig. 14 shows a schematic representation of a preferred method and the corresponding equipment according to the present invention useful for making the mechanical closure tape 10 obtainable from the precursor of Fig. 2. The equipment comprises 6 unwind stands 50 - 55, respectively, feeding the backing 11 bearing a layer 12 (unwind stand 50), the fingerlift 15 (unwind stand 51), the mechanical fastening means 14 (unwind stand 52), the covering film 13 (unwind stand 53), the first one-sided adhesive layer 16 (unwind stand 54) and the second one-sided adhesive layer 17 (unwind stand 55), respectively, in the order given into a lamination device comprising two lamination rolls 56 and 57. The mechanical closure tape 10 which is thus obtained in a single lamination step, can subsequently be wound up on roll 58 for storage.

The mechanical closure tapes 10 according to the present invention or their precursors, respectively, which are shown in Fig. 4, 6, 8, 10, 12, 14b and 15, respectively, or further embodiments can be obtained similarly. The mechanical closure tape of Fig. 8 requires double-folding of the one-sided adhesive tape 16 which can be performed by a conventional folding unit comprising one or more creasing wheels and one or more folding rollers and/or folding bars. Likewise, the proximal end of the precursor of the mechanical closure tape of Fig. 10 needs to be folded back onto itself prior to attaching the closure tape 10 to the edge portion of the diaper 1.Manufacturing of the mechanical adhesive tape according to Fig. 14b requires two seperate unwind stands for the two parts 11a and 11b, respectively, of the backing. When preparing the mechanical closure tape 10 according to Fig. 14a, the backing 11 bearing the adhesive layer 12 is passed through a rotary cutter to provide the incisions 25 and the stretchable elastic film 13a is laminated to the adhesive layer 12 while the backing 11 and the adhesive layer are still supported by the rotary cutter in order to maintain the integrity of the backing 11.

Absorbent articles such as diapers are mass products, and the closure tape 10 used therein must be
- user-friendly and easy to apply, and
- easy and comparatively cheap to manufacture.

The mechanical closure tape 10 of the present invention meets these requirements to a particularly high degree. The exposed part of adhesive layer 16a provides, in combination with the mechanical fastening means 14, a two-point closure system which reliably secures the diaper to the wearer's body during use, and it furthermore provides an anti-flagging and a disposal feature. The mechanical closure tape 10 is easy to manufacture. The mechanical closure tape which is obtainable from the precursor of Fig. 2, for example, can be obtained in a single lamination step which allows for a particularly high line speed of, for example, 100 m/min of the laminate or more.

Contrary to this, the manufacture of the closure tapes disclosed in EP 0,780,109 requires strip-coating and/or the use of double-coated adhesive tapes. Coating steps require subsequent drying of the tape construction which tends to decrease the manufacturing speed and to considerably increase the dimensions of the equipment because of the need for a drying station. Double-coated adhesive tapes require at least one release liner which has to be removed prior to the lamination step and discarded. Double-sided adhesive tapes increase the thickness of the part of the resulting closure tape where they are present. The resulting closure tapes are particularly inhomogenous in thickness which makes it difficult to wind them up in a stable roll form.

## Claims

1. A mechanical closure tape (10) for an absorbent article, particularly for a disposable diaper (1), for the fastening of the article on the body of a person, the mechanical closure tape (10) being attachable to the outside surface (3) of the diaper (1) through its manufacturer's end (19) and comprising a backing (11) bearing an adhesive layer (12), optionally a covering film (13) and/or a stretchable elastic film (13a), a mechanical fastening means (14), optionally a fingerlift (15) and a one-sided adhesive tape (16) having a backing (16b) bearing an adhesive layer (16a), the adhesive tape (16) being attachable to the inside surface (2) of the diaper and/or to the connective portion (21) of the closure tape (10) by means of the adhesive layer (16a), by means of the adhesive layer (12) and/or by means of one or more one-sided further adhesive tapes so that the adhesive layer (16a) is at least partially exposed above the inside surface (2) of the diaper.

2. Mechanical closure tape according to claim 1 wherein the distal end of the adhesive tape (16) is attached to the adhesive layer (12) of the closure tape (10).

3. Mechanical closure tape according to claims 1 or 2 wherein the proximal end of the adhesive tape (16) is attached to the inside surface of the diaper with a one-sided adhesive tape (17).

4. Mechanical closure tape according to claim 3 wherein the one-sided adhesive tape 17 comprises a backing (17b) bearing an adhesive layer (17a).

5. Mechanical closure tape according to claim 4 wherein the backing (17b) comprises a mechanical fastening element (17c).

6. Mechanical closure tape according to claims 1 or 2 wherein the proximal end of the covering film (13) overlaps with the distal end of the adhesive tape (16).

7. Mechanical closure tape according to claims 1 or 2, wherein the proximal end of the adhesive tape (16) is folded back onto its backing (16b) to allow for adhering of the proximal end of the adhesive tape (16a) to the inside surface (2) of the absorbent article.

8. Mechanical closure tape according to claims 1 or 2 wherein the pressure sensitive adhesive tape (16) is folded twice on itself so that it has a first part adhered to the pressure sensitive adhesive layer (16), a second part the pressure sensitive adhesive layer (16a) of which being exposed and a third part between the first and second part of the adhesive tape (16) so that the adhesive layer (16a) of the third part contacts the backing (16b) of the first part of the adhesive tape (16).

9. Mechanical closure tape according to claims 1 or 2 where the distal end of the adhesive tape (16) is secured to the adhesive layer (12) by means of a one-sided pressure sensitive adhesive tape (18) comprising a backing (18b) bearing a backing (18a).

10. Mechanical closure tape according to claims 1or 2 comprising a stretchable elastic film (13a) and exhibiting one or more incisions (25) in the area of the stretchable eleastic film (13a) with at least one of the incisions (25) extending in vertical direction through the backing (11) and the adhesive layer (12) and in machine direction over the full width of the backing (11).

11. Absorbent article comprising a mechanical closure tape according to claims 1 - 10.

12. Prelaminated mechanical closure tape in a stable roll from which the mechanical closure tape for an absorbent article according to claims 1 - 10 can be cut.

13. Method for preparing a closure tape (10) according to claims 1 - 10, comprising laminating the backing (11) bearing adhesive layer (12), optionally the covering film (13), the mechanical fastening means (14), optionally the fingerlift (15), the one-sided adhesive tape (16) and, optionally, one or more further one-sided adhesive tapes.

14. Method according to claim 13 wherein the lamination is performed in one lamination device.
